**Europäisches Patentamt**

**European Patent Office**

**Office européen des brevets**

(19)

(11) Publication number : **0 465 550 B1**

## EUROPEAN PATENT SPECIFICATION

(12)

(45) Date of publication of patent specification :
15.12.93 Bulletin 93/50

(51) Int. Cl.⁵ : **A61K 9/10,** A61K 31/195,
A61K 31/20, A61K 37/02

(21) Application number : 90905734.1

(22) Date of filing : 26.03.90

(86) International application number :
PCT/SE90/00190

(87) International publication number :
WO 90/11753 18.10.90 Gazette 90/24

(54) PHARMACEUTICAL LIPID COMPOSITION FOR PARENTERAL NUTRITION.

(30) Priority : 30.03.89 US 330754

(43) Date of publication of application :
15.01.92 Bulletin 92/03

(45) Publication of the grant of the patent :
15.12.93 Bulletin 93/50

(84) Designated Contracting States :
AT BE CH DE DK ES FR GB IT LI LU NL SE

(56) References cited :
WO-A-88/01861
US-A- 4 254 147
US-A- 4 526 793
US-A-82 541 473
Medical Hypotheses, vol. 13, 1984 Mark F. Mc Carty et al; " Rationales for micronutrient supplementation in diabetes", see page 139 - page 151 Page 142 - 144
Lipids, vol.22, no.3, 1987 Manuela Martinez et al: "Effects of Parenteral Nutrition with High Doses of Linoleate on the Developing Human Liver and Brain"

(56) References cited :
Journal of Parenteral and Enteral Nutrition, Vol. 8, No. 1, 1984 H. Bohles M.D. et al: "Improved N-retention during L-Carnitine-Supplemented Total Parenteral Nutrition" Lipids, Vol. 23, No. 4, 1988 Larry D. Lawson et al: "Triacylglycerol Structure of Plant and Fungal Oils Containing y-Linolenic Acid" Oléagineux, Vol. 42, 1987 J.M. Muderhwa et al: "Réparartition interne/externe des acides gras des triglycérides de quelques huiles gamma linoléniques"

(73) Proprietor : KABIVITRUM AB
S-112 87 Stockholm (SE)

(72) Inventor : LINDMARK, Lars
Ejdervägen 8
S-230 11 Falsterbo (SE)
Inventor : WENNBERG, Annika
Skogsvägen 7
S-181 41 Lidingö (SE)

(74) Representative : Onn, Thorsten et al
AB STOCKHOLMS PATENTBYRA Box 23101
S-104 35 Stockholm (SE)

EP 0 465 550 B1

## Description

This invention relates to a lipid composition for parenteral nutrition of patients in the need thereof. The invention further relates to the therapeutic use of said lipid composition for anabolic purposes i.e. biosynthesis of cell membranes and prostanoids, in that it relates to the suitability of the lipid components of said lipid composition.

Refined vegetable oils such as soybean oil and safflower oil, prepared for human use in the form of oil-in-water emulsions, for example according to US Patent No. 4,101,673, have been used successfully as intravenous energy sources in various pathological situations where adequate oral food intake is impossible or contraindicated.

Besides constituting a high density energy source with minimal osmotic effect when infused into the blood stream, the emulsified vegetable oils provide fatty acids, some of which are essential for the biosynthesis of cell membranes and prostanoids.

Vegetable oils used in existing lipid compositions for parenteral nutrition, such as soybean oil and safflower oil contain high amounts of linoleic acid (18:2 n-6).

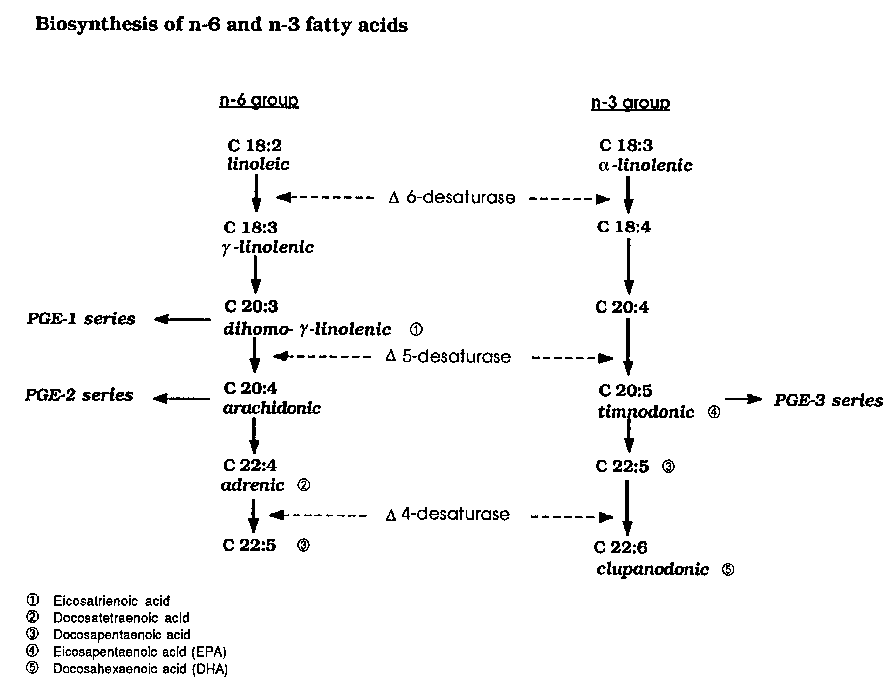

It has been shown that administration of the relatively high contents of linoleic acid (18:2 n-6) in total parenteral nutrition to premature and full-term infants influences the fatty acid composition of cell membranes in brain tissue with a significant increase in linoleic acid and a concomitant decrease in elongation/desaturation products of this essential fatty acid, for example arachidonic acid (20:4 n-6) which normaly increases during the maturation process of the brain. Thus the essential fatty acid status in growing tissues respond rapidly to the dietary content of these fatty acids (Martinez and Ballabriga: Lipids, 22; 133-138 (1987). During the gestational period there is a selective transport of elongation/desaturation products of linoleic acid and alpha-linolenic acid across the placental barrier. The conversion of the essential long-chain polyunsaturated fatty acids linoleic acid (18:2 n-6) and alpha-linolenic acid (18:3 n-3) to their longer-chain derivatives, which are substrates for prostanoid biosynthesis, takes place through alternating desaturation and elongation reactions. It is generally believed that the same enzymes catalyze equivalent steps in the n-6 and n-3 fatty acid families, as is shown in the drawing. The delta-6-desaturase is the first and rate limiting enzyme in this chain process where linoleic acid is converted into gamma-linolenic acid (18:3 n-6). An excess of linoleic acid itself and/or alpha-linolenic acid inhibits this reaction.

In the previously mentioned study, administration of high amounts of linoleic acid in parenteral nutrition gave significant decreases in the higher homologues of the n-6 series in liver phospholipids, the liver being an important organ for these metabolic processes. Experimental data also suggest that substrate inhibition occurs. Furthermore a decreased production of prostaglandin $E_1$ from dihomo-gamma-linolenic acid, the elongation product of gamma-linolenic acid, was seen in infants receiving parenteral nutrition with existing products compared to controls. The advantage of an improvement of existing lipid compositions for parenteral nutrition by inclusion of a lipid component containing gamma-linolenic acid is evident. This is already known, for example through US patent No. 4,526,793.

However, the nature of the gamma-linolenic acid-containing lipid component in its ability to produce increased amounts of dihomo-gamma-linolenic acid is essential.

Oil-in-water emulsions given in parenteral nutrition therapy are present in the blood stream in the form of chylomicron-like particles. The triglyceride portion of these particles is hydrolysed by the lipoprotein lipase at the vascular endothelial wall. Lipoprotein lipase preferentially hydrolyses fatty acids in the 1- and 3 positions of the glycerol moiety. The fatty acids then enter the cells and are used as an energy source through beta-oxidation, or are stored. Infusion of energy substrates also inhibits the release of endogenous fatty acids from, for example, adipose tissues. The remnant particles, after the lipoprotein lipase hydrolysis, contain some monoglycerides of which a substantial portion is expected to have the fatty acid in the 2-position. These remnant particles are taken up by the liver and their contents is used for metabolic purposes, such as production of desaturation/elongation products of fatty acids and reexported in the form of lipoproteins such as VLDL and LDL. Therefore, in order to ascertain the availability of gamma-linolenic acid for anabolic purposes, such as biosynthesis of cell membranes and prostaglandins, the preferred triglyceride containing gamma-linolenic acid should have said fatty acid in the 2-position. Oil from seeds of plants of the genus Borago contains triglycerides with high amounts of gamma-linolenic acid (20-30 % of total fatty acids) and 55-60 % of the gamma-linolenic acid is found in the 2-position. In oil from the genus Ribes as described in the US Pat. No. 4,526,793, with a content of gamma-linolenic acid below 20 % of total fatty acids, only 36-38 % of the gammalinolenic acid is found in the 2-position (Lawson and Hughes, Lipids 23: 313-317 (1988); Muderhwa et al, Oleagineux 42: 207-211 (1987)). According to this invention, the therapeutic use of the lipid composition for parenteral nutrition for anabolic purposes is ascertained by introducing triglycerides from the oil of the genus Borago, preferably Borago Officinalis, as a lipid component with high bioavailability of gamma-linolenic acid.

The other components in the lipid composition of this invention preferably include purified vegetable oils which are known to be well tolerated when supplied intravenously in the form of oil-in-water emulsions, such as soybean oil purified according to US patent 4,101,673. Soybean oil is preferred because its contains both linoleic acid (18:2 n-6) and alpha-linolenic acid (18:3 n-3) which are essential to humans. A few cases of alpha-linolenic acid deficiency have been described in the literature and alpha-linolenic acid is a precursor for the desaturation/elongation product eicosapentaenoic acid (20:5 n-3) from which important prostanoids are synthetized.

It is well established that L-carnitine acts as a carrier of fatty acids with more than ten carbon atoms across the inner mitochondrial membrane. By means of specific enzymes (acyl and acetyl transferases and translocases), L-carnitine permits the reversible transfer of activated fatty acids from the cytosol into the sites of beta-oxidation in the mitochondria. Carnitine deficiency is accompanied by disorders in lipid metabolism and with lipid storage in muscles and liver, which, in most cases, is improved by treatment with L-carnitine.

It has been reported that patients receiving TPN for longer periods, especially premature infants and neonates, develop subnormal carnitine levels in plasma. However, an excessive carnitine supply such as that described in US patent 4,254,147 can inhibit carnitine-acyl-transferase and the utilization of fatty acids. It is therefore preferable to include amounts of L-carnitine equivalent to those normally present in human milk, which are known to promote oxidation of fatty acids as well as ketogenesis which are required for the survival of the newborn infant. When 1.5 mg/kg/day of L-carnitine was given intravenously, lipolysis and oxidation of fatty acids were more effective; there was an increase in the energy gain from exogenous fat and an improvement in nitrogen balance (Böhles et al, JPEN 8: 9-13 (1984)). It is also evident that the supply of L-carnitine should be related to the supply of fatty acids, preferably between 0.5 and 5 mg per g of fatty acids.

The sole drawing figure shows a scheme of the biosynthesis of the n-6 and n-3 fatty acids.

According to one aspect of the invention, there is provided a pharmaceutical composition of refined vegetable oils of which one of the oils is extracted from the seeds of the genus Borago, said oil having anabolic properties advantageous for treatment of patients in the need of parenteral nutrition.

The composition is further characterized by its content of L-carnitine in relation to its lipid content.

According to another aspect of the invention, the vegetable oils are mixed, purified and emulsified to provide an oil-in-water emulsion suitable for parenteral administration.

Best and Various Modes For Carrying Out Invention

The recommendation for the supply of essential fatty acids to healthy persons is around 6-10 % of the total energy intake for linoleic acid (18:2 n-6) and 0.5-1 energy percent for alpha-linolenic acid. The requirement for gamma-linolenic acid is positively correlated to the supply of both linoleic acid and alpha-linolenic acid. The supply should be sufficient to increase production of di-homo-gamma-linolenic acid but should not significantly alter the n-6/n-3 ratio of the supply of fatty acids. The references in the specification and drawing regarding the fatty acids, such as C18: 3n-6 for gamma-linolenic acid are based on the IUPAC-IUB Commission on Biochemical nomenclature for the notation of fatty acids to show the number of carbon atoms and double bonds, and the location of the double bonds.

A suitable dose of gamma-linolenic acid from the preferred oil from seeds of the genus Borago in the lipid composition is therefore between 2 and 30 g/day, about 2 g being the minimum requirement for adult healthy persons. A suitable dose of L-carnitine to promote the utilization of long-chain fatty acids, hydrolysed from supplied triglycerides by lipoprotein lipase, as energy substrates is related to the amount of fatty acids liberated and is preferrably correlated to the content of these substances in human milk (20 mg of L-carnitine and 30-40 g of fat per liter of human milk), bearing in mind that carnitine supplied in human milk appears to be more efficiently utilized. Between about 0.5 and about 5 mg L-carnitine per g of supplied fatty acids is therefore considered as an adequate dose.

The lipid composition of this invention is particularly suitable for parenteral nutrition of patients in the need thereof for biosynthesis of cell membranes and prostanoids This especially is the case for infants.

Thus, the lipid composition of the invention preferably comprises:

a) a lipid mixture of refined and purified vegetable oils, containing about 10 to about 30 weight percent of oil from the genus Borago, which contains gamma-linolenic acid in the form of triglycerides, said gamma-linolenic acid constituting from about 2 to about 10 weight percent of the lipid mixture;

b) L-carnitine in an amount of about 0.1 to about 0.5 grams per 100 grams of the lipid mixture;

c) one or more auxiliary components, selected among emulsifying agents, stabilizing agents, agents for regulating the osmotic pressure, preserving agents, nutritive agents, trace elements and vitamins, and

d) an aqueous phase in such an amount that the finished emulsion contains about 50 to about 350 grams of lipid mixture per 1000 ml of emulsion.

The further components of the lipid mixture under a) may be vegetable oils containing long-chain polyunsaturated fatty acids in the form of triglycerides having a ratio n-6/n-3 from 6 to 10. Preferably this vegetable oil consists of soybean oil.

The auxiliary components under c) are those that are conventionally used in fat emulsions for parenteral administration in the amounts conventionally used, such as those disclosed in U.S. Patent 3,169,094. Thus, the emulsifying agent may consist of one or more of the pharmaceutically acceptable agents conventionally used, such as egg yolk phospholipids, soybean phospholipids or various emulsifiers of a synthetic origin. Typical amounts are about 0.50 to about 3% by weight of the emulsion. One or more emulsifiers can be used in the emulsion.

Regarding the stabilizing agents, a number of such agents are known to those skilled in the art. These agents should stabilize the emulsion at its preparation, sterilization and storage, and also against the type of breakdown called "creaming", which may occur if the emulsion is mixed with blood, plasma or serum from sick patients.

The agents for regulating the osmotic pressure are important for making the emulsion isotonic with the blood. As an example of such agents can be mentioned glycerol, which also serves as a source of energy.

Various nutritive agents, trace elements and vitamins for use in emulsions for nutrition are well-known. As two important groups of nutritive agents can be mentioned amino acids and carbohydrates. The vitamins used may be both fat-soluble and water-soluble, and will then be present in the emulsion in the oil phase and the aqueous phase, respectively.

The emulsion is prepared in accordance with well-known techniques. Usually the fats, the aqueous phase, the emulsifier and the other constituents are mixed, and the mixture is thereafter homogenized in a suitable apparatus. After homogenization to a suitable particle size of the fat phase, the emulsion is filled into suitable bottles and sterilized. The emulsion may also be sterilized first and thereafter filled into bottles under sterile conditions.

An emulsion which is to be injected into the blood vessels must have a particle size below the size of the blood corpuscles, i.e. below about 6 $\mu$m. Preferably the particle size should be below about 4 $\mu$m, and the main fraction of the particles should have a size below about 1 $\mu$m, preferably then between 0.05 and 0.5 $\mu$m. It is well-known to a person skilled in the art to prepare an emulsion having a suitable particle size.

It is important that the components used for the emulsion are of a pharmaceutically irreproachable quality from the start and that this quality is upheld through the preparation process. This is of special importance for the fats and certain emulsifiers, such as phospholipids, which must be purified, for instance by suitable extraction and precipitation processes, and then be protected from oxygen, to prevent the formation of oxidation products. The water used for the aqueous phase must also be sterile and free from pyrogens. All this is well-known in the art.

The following examples are given to illustrate the preferred embodiments of the invention.

Example 1

A oil mixture for parenteral nutrition is prepared from refined and purified Borage oil and soybean oil.

The crude Borage oil is heated to 90°C and then treated with 1.0 ml concentrated phosphoric acid/kg oil during 5 minutes. The oil is then treated with 4 M NaOH, in 50 % excess according to the content of free fatty acids in the crude oil and the added phosphoric acid, for 20 minutes under nitrogen gas atmosphere. The soap stock is separated at 10.000 rpm with a separator at an oil flow of approximately 1 l/minute. The separated oil is washed with 90°C pyrogen-free water until a clear water phase is obtained (about 5 times). The washed oil is dried under vacuum (<665 Pa) at 80-90°C. Traces of metals present in the oil are chelated by adding 1.0 ml of citric acid solution (25 g citric acid dissolved in 95 % ethyl alcohol to 100 ml) per kg of oil, at 90°C during 30 minutes, under vacuum. Then, 3 % (w/w) bleaching earth and activated carbon in a 3:1 ratio is added at 90°C during 30 minutes and under vacuum. The oil is then cooled below 60°C and filtered by over-pressure in a nitrogen gas atmosphere.

The Borage oil and a commercially refined, bleached, and deodorized soybean oil are mixed proportionally to the actual gamma-linolenic acid content in Borage oil, so as to give a concentration of 3-4 % gamma-linolenic acid in the resulting mixture.

Purification of the oil mixture is carried out according to U.S. Pat. No. 4,101,673.

Analysis of a purified mixture of Borage oil and Soybean oil:

| | |
|---|---|
| Iodine value | 137 |
| Free fatty acids (mmol/kg) | < 1.0 |
| Saponification value | 193 |
| Peroxides (mmol/kg) | 0.0 |

4

Refractive index $N_D^{25}$            1.743

Heavy metals (ppm)            <10

Fatty acid composition (weight percent of total fatty acids) :

| | | |
|---|---|---|
| C16:0 | Palmitic acid | 10.2 |
| C18:0 | Stearic acid | 3,9 |
| C18:1 | Oleic acid | 20.7 |
| C18:2 | Linoleic acid | 51.9 |
| C18:3 n-3 | Alpha-linolenic acid | 6.2 |
| C18:3 n-6 | Gamma-linolenic acid | 3.5 |

A formula for a parenteral nutritional emulsion is prepared by the procedure described in U.S. Pat. No. 4,101,673, using the following ingredients:

| | |
|---|---|
| Purified mixture of Borage oil and soybean oil | 200 g |
| Egg phospholipids | 12 g |
| Glycerol | 22.5 g |
| L-carnitine (added in the glycerol/water solution) | 0.4 g |
| Sodium hydroxide for pH adjustment to about pH 8 | |
| Sterile water for injection ad | 1000 ml |

Analysis of the Borage oil-soybean oil emulsion gave:

| | |
|---|---|
| Free fatty acids (mmol/l) | <5 |
| Droplet size (% droplets>1μm of all droplets>0.5 μm) | <5 |

Fatty acid composition (weight% of total fatty acids) :

| | | |
|---|---|---|
| C16:0 | Palmitic acid | 10 |
| C18:0 | Stearic acid | 4 |
| C18:1 | Oleic acid | 20 |
| C18:2 | Linoleic acid | 50 |
| C18:3 n-3 | Alpha-linolenic acid | 7 |
| C18:3 n-6 | Gamma-linolenic acid | 3 |

The content of gamma-linolenic acid was 7 g/l

Example 2

A 20 % nutritional oil-in-water emulsion for parenteral use, prepared according to the procedures outlined in example 1, was infused via a peripheral vein intravenously, to each of 4 dogs (2 male + 2 female Beagles, 30 weeks of age) over a 6-hour period at a rate of 45 ml/kg/hr for a period of 28 consecutive days. In the same study 4 other dogs (same species, sex ratio and age) received identical amounts of a commercially available fat emulsion with 20 % soybean oil and an egg-yolk phospholipid emulsifier, and another 4 dogs (same species, sex ratio and age), received an identical volume of an electrolyte solution for the same period.

Blood samples were obtained in heparinized tubes before starting the infusions and on the day after the complete infusion period after an over-night fast. Plasma was separated by centrifugation and frozen. Specimens of liver tissue were obtained at autopsy and frozen immediately. In the plasma and liver tissue samples analysis of total fatty acid content and fatty acid distribution was performed using gas-liquid chromatography after extraction of lipids according to known procedures. Total carnitine content was estimated in the plasma samples by a radioenzymatic assay.

The results are depicted in table 1 regarding values from the analysis of plasma and in table 2 regarding values from the analysis of the liver.

From the results it is evident that the nutritional oil-in-water emulsion prepared as outlined in example 1 possesses the properties described in the invention. A substantial increase in the elongation product of gamma-linolenic acid, dihomo-gamma-linolenic acid (20:3 n-6), was noted both in plasma and liver after administration of the emulsion described in example 1, compared to a commercially available soybean oil emulsion. The content of arachidonic acid was also normalized in liver tissue when compared to the values obtained in the control group and the group given soybean oil emulsion.

The administration of the oil-in-water emulsion described in example 1 contained 0.4 g/l of L-carnitine, which amount was sufficient to cause a substantial increase in plasma concentration of carnitine. In the dogs that received the soybean oil emulsion a decrease in total plasma carnitine was noted.

```
Table 1.
Total fatty acid content, distribution of specific
fatty acids as % of total fatty acids and total
carnitine concentration in plasma before and after
intravenous administration in dogs of an oil-in-water
emulsion described in example 1, a commercially
available soybean oil emulsion and an electrolyte
solution.
```

| | Borage oil-soybean oil emulsion (acc. to example 1) n=4 | | Soybean oil emulsion n=4 | | Electrolyte solution n=4 | |
|---|---|---|---|---|---|---|
| | Before | After | Before | After | Before | After |
| Total fatty acids mg/ml (Mean±SD) | 2.8±0.4 | 4.9±0.4 | 2.8±0.2 | 4.4±0.4 | 2.9±0.4 | 2.6±0.5 |
| Total carnitine μmol/l (Mean±SD) | 15.3±0.6 | 39.0±5.0 | 15.2±1.0 | 10.5±1.1 | 12.8±1.8 | 13.4±2.7 |
| Fatty acid distribution (% of total fatty acids): | | | | | | |
| Linoleic acid (18:2 n-6) | 23.5 | 25.5 | 22.5 | 26.6 | 22.8 | 25.9 |
| Gamma-linolenic acid (18:3 n-6) | 0.1 | 0.6 | 0.1 | 0.2 | 0.1 | 0.1 |
| Dihomo-gamma-linolenic acid (20:3 n-6) | 1.1 | 2.0 | 1.1 | 1.1 | 1.2 | 1.1 |
| Arachidonic acid (20:4 n-6) | 18.6 | 12.1 | 19.3 | 11.0 | 18.7 | 15.0 |

Table 2

Total fatty acid content and distribution of specific fatty acids as % of total fatty acids in liver tissue after intravenous administration in dogs of an oil-in-water emulsion discribed in example 1, a commercially available soybean oil emulsion and an electrolyte solution. Fatty acid distribution in % of the values obtained with the electrolyte solution within brackets.

| | Electrolyte solution n=4 | Soybean oil emulsion n=4 | Borage oil-soybean oil emulsion (acc. to example 1) n=4 |
|---|---|---|---|
| Total fatty acid content g/100 g wet weight (Mean±SD) | 2.7±0.2 | 6.6±1.7 | 3.5±0.4 |
| Fatty acid distribution in % of total fatty acids: | | | |
| Linoleic acid (18:2 n-6) | 17.1 | 40.0 (+134%) | 26.4 (+54%) |
| Gamma-linolenic acid (18:3 n-6) | 0.17 | 0.53 (+212%) | 0.63 (+270%) |
| Dihomo-gamma-linolenic acid (20:3 n-6) | 1.32 | 0.96 (-27%) | 3.16 (+140%) |
| Arachidonic acid (20:4 n-6) | 15.89 | 7.87 (-50%) | 14.63 (-8%) |

Example 3

The 20 % oil-in-water emulsion for parenteral use described in example 1 was administered intravenously to 24 healthy male subjects, each of which received 250 ml over a period of four hours (63 ml/h). In a cross-over design the same healthy persons received an identical dose of a commercially available 20 % soybean oil emulsion with egg yolk phospholipids as emulsifier.

Blood was collected in heparinized tubes pre and post infusion and centrifugated. Plasma was analysed for the content of gamma-linolenic acid (18:3 n-6), dihomo-gamma-linolenic acid (20:3 n-6), arachidonic acid (20:4 n-6) and free and total carnitine. The differences post-preinfusion were calculated and the treatment related differences evaluated. The values are given in table 3. Highly significant differences in the post-preinfusion concentrations of gamma-linolenic acid and free and total carnitine were noted between the emulsions. The content of the elongation product of gamma-linolenic acid, dihomo-gamma-linolenic acid was increased after a single infusion for 4 hours of the oil-in-water emulsion described in example 1 compared to the control emulsion (p=0.08), which provides evidence for the metabolic advantage of the invented pharmaceutical composition.

8

Table 3

Comparison of differences post-preinfusion in plasma concentrations of specific fatty acids and carnitine after intravenous administration of the oil-in-water emulsion described in example 1 and commercially available soybean oil emulsion in 24 healthy subjects ($\mu$mol/l Mean[+]SD).

| | Borage oil-soybean oil emulsion | Soybean oil emulsion | P |
|---|---|---|---|
| Gamma-linolenic acid (18:3 n-6) | 239±133 | 0.3±11.3 | < 0.001 |
| Dihomo-gamma-linolenic acid (20:3 n-6) | 11.4±24.5 | 1.7±19.6 | 0.08 |
| Arachidonic acid (20:4 n-6) | 15.4±67.7 | 12.6±79.0 | 0.86 |
| Free carnitine | 12.3±4.9 | − 3.9±4.0 | < 0.001 |
| Total carnitine | 18.0±6.8 | − 0.9±6.8 | < 0.001 |

Example 4

The borage oil and a commercially refined, bleached and deodorized soybean oil are mixed proportionally to the actual gamma-linolenic acid content in Borage oil so as to give the concentration of 3-4 % gamma-linolenic acid in the resulting mixture. Purification of the oil mixture is according to US Pat No. 4,101,673.

Analysis of a purified Borage-Soybean oil mixture:

| | |
|---|---|
| Iodine value | 137 |
| Free fatty acids (mmol/kg) | < 1.0 |
| Saponification value | 193 |
| Peroxides (mmol/kg) | 0.1 |
| Refractive indes $N_D^{25}$ | 1.743 |
| Heavy metals (ppm) | <10 |

Fatty acid composition (% of total fatty acids)

| C16:0 | Palmitic acid | 10.2 |
|---|---|---|
| C18:0 | Stearic acid | 3.9 |
| C18:1 | Oleic acid | 20.7 |
| C18:2 n-6 | Linoleic acid | 51.9 |
| C18:3 n-3 | Alpha-linolenic acid | 6.2 |
| C18:3 n-6 | Gamma-linolenic acid | 3.5 |

A formula for a parenteral oil-in-water emulsion is prepared by the procedure described in U.S. Pat. No. 4,101,673 using the following ingredients :

```
Purified mixture of Borage and Soybean oils       300    g
Eggyolkphospholipids                               12    g
Glycerol                                         22.5   g
L-carnitine (added in glycerol/water solution)    0.4  g
Sodium hydroxide to pH adjustment to about pH 8
Sterile water for injection ad                   1000   ml
```

Analysis of a Borage-Soybean oil emulsion:

```
Free fatty acids (mmol/1)                  <5
Droplet size (% droplets >1 μm of all
droplets >0.5 μm)                          <5
```

Fatty acid composition (% of total fatty acids)

```
C16:0        Palmitic acid                  10
C18:0        Stearic acid                    4
C18:1        Oleic acid                     21
C18:2 n-6    Linoleic acid                  51
C18:3 n-3    Alpha-linolenic acid            6
C18:3 n-6    Gamma-linolenic acid            3
```

The content of gamma-linolenic acid was 9,9 g/l

Example 5

The borage oil and a commercially refined, bleached and deodorized soybean oil are mixed proportionally to the actual gamma-linolenic acid content in Borage oil so as to give the concentration of 7-8 % gamma-linolenic acid in the resulting mixture. Purification of the oil mixture is according to US Pat. No. 4,101,673.

Analysis of a purified Borage-Soybean oil mixture:

| | |
|---|---|
| Iodine value | 131 |
| Free fatty acids (mmol/kg) | <1.0 |
| Saponification value | 190 |
| Peroxides (mmol/kg) | 0.1 |
| Refractive index $N_D^{25}$ | 1.474 |
| Heavy metals (ppm) | <10 |

Fatty acid composition (% of total fatty acids)

```
C16:0        Palmitic acid                10.3
C18:0        Stearic acid                  3.6
C18:1        Oleic acid                   18.8
C18:2 n-6    Linoleic acid                49.8
C18:3 n-3    Alpha-linolenic acid          4.8
C18:3 n-6    Gamma-linolenic acid          7.4
```

EP 0 465 550 B1

A formula for a parenteral oil-in-water emulsion is prepared by the procedure described in U.S. Pat. No. 4,101,673 using the following ingredients :

```
Purified mixture of Borage and soybean oils      100    g
Eggyolkphospholipids                              12     g
Glycerol                                          22.5   g
L-carnitine (added in glycerol/water solution)    0.4    g
Sodium hydroxide to pH adjustment to about pH 8
Sterile water for injection ad                    1000   ml
```

Analysis of the Borage-Soybean oil emulsion:

```
Free fatty acids (mmol/l)                   <5
Droplet size (% droplets >1 µm of all
droplets >0.5 µm)                           <5
```

Fatty acid composition (% of total fatty acids)

```
C16:0       Palmitic acid              10
C18:0       Stearic acid               4
C18:1       Oleic acid                 19
C18:2 n-6   Linoleic acid              50
C18:3 n-3   Alpha-linolenic acid       5
C18:3 n-6   Gamma-linolenic acid       7
```

The content of gamma-linolenic acid was 7.4 g/l

## Claims

**Claims for the following Contracting States : AT, BE, CH, DE, DK, FR, GB, IT, LI, LU, NL, SE**

1. A composition for parenteral nutrition, consisting of an oil-in-water emulsion, which comprises:
   a) a lipid mixture of refined and purified vegetable oils, containing 10 to 30 weight percent of oil from the genus Borago, which contains gamma-linolenic acid as triglycerides, said gamma-linolenic acid constituting 2 to 10 weight percent of the lipid mixture;
   b) L-carnitine in an amount of 0.1 to 0.5 g per 100 g of the lipid mixture;
   c) at least one auxiliary component, selected from the group of emulsifying agents, stabilizing agents, agents for regulating the osmotic pressure, preserving agents, nutritive agents, trace elements and vitamins, and
   d) an aqueous phase in such an amount that the finished emulsion contains 50 to 350 g of lipid mixture per 1000 ml of emulsion.

2. A composition in accordance with claim 1, wherein the lipid mixture further comprises at least one vegetable oil which contains long-chain polyunsaturated fatty acids as triglycerides having a ratio n-6/n-3 of 6 to 10.

3. A composition in accordance with claim 2, wherein the vegetable oil is soybean oil.

11

4. The composition of claim 1 wherein said emulsion has a particle size below 8 µm.

5. The composition of claim 1 wherein said emulsion has a particle size below 4 µm with the main fraction of particles below 1µm.

6. The composition of claim 1 wherein said emulsifying agent includes egg yolk phospholipids or soybean phospholipids or both.

7. The composition of claim 1 wherein said agent for regulating the osmotic pressure includes glycerol.

8. The composition of claim 1 wherein said nutritive agents include amino acids or carbohydrates or both.

**Claims for the following Contracting States : ES, GR**

1. A method for the preparation of a composition for parenteral nutrition, consisting of an oil-in-water emulsion, which comprises:
   a) a lipid mixture of refined and purified vegetable oils, containing 10 to 30 weight percent of oil from the genus Borago, which contains gamma-linolenic acid as triglycerides, said gamma-linolenic acid constituting 2 to 10 weight percent of the lipid mixture;
   b) L-carnitine in an amount of 0.1 to 0.5 g per 100 g of the lipid mixture;
   c) at least one auxiliary component, selected from the group of emulsifying agents, stabilizing agents, agents for regulating the osmotic pressure, preserving agents, nutritive agents, trace elements and vitamins,
   and
   d) an aqueous phase in such an amount that the finished emulsion contains 50 to 350 g of lipid mixture per 1000 ml of emulsion,
   characterized in that the components a) to c) are mixed, and the mixture is thereafter homogenized.

2. A method in accordance with claim 1, wherein the lipid mixture further comprises at least one vegetable oil which contains long-chain polyunsaturated fatty acids as triglycerides having a ratio n-6/n-3 of 6 to 10.

3. A method in accordance with claim 2, wherein the vegetable oil is soybean oil.

4. The method of claim 1 wherein said emulsion is homogenized to a particle size below 8 µm.

5. The method of claim 1 wherein said emulsion is homogenized to a particle size below 4 µm with the main fraction of particles below 1 µm.

6. The method of claim 1 wherein said emulsifying agent includes egg yolk phospholipids or soybean phospholipids or both.

7. The method of claim 1 wherein said agent for regulating the osmotic pressure includes glycerol.

8. The method of claim 1 wherein said nutritive agent include amino acids or carbohydrates or both.

**Patentansprüche**

**Patentansprüche für folgende Vertragsstaaten : AT, BE, CH, DE, DK, FR, GB, IT, LI, LU, NL, SE**

1. Zusammensetzung für die parenterale Ernährung, bestehend aus einer Öl-in-Wasser-Emulsion, enthaltend
   a) ein Lipidgemisch von raffinierten und gereinigten pflanzlichen Ölen, enthaltend 10 bis 30 Gew.-% Öl der Gattung Borago, welches gamma-Linolensäure als Triglyceride enthält, wobei die gamma-Linolensäure 2 bis 10 Gew.-% des Lipidgemisches ausmacht,
   b) L-Carnitin in einer Menge von 0,1 bis 0,5 g pro 100 g des Lipidgemisches,
   c) mindestens eine Hilfskomponente, ausgewählt aus der von Emulgatoren, Stabilisierungsmitteln, Mitteln zur Regulierung des osmotischen Druckes, Konservierungsmitteln, Nährstoffen, Spurenelementen und Vitaminen gebildeten Gruppe und

d) eine wässrige Phase in einer Menge, daß die fertige Emulsion 50 bis 350 g Lipidgemisch pro 1000 ml Emulsion enthält.

2. Zusammensetzung nach Anspruch 1, bei der das Lipidgemisch weiterhin mindestens ein pflanzliches Öl umfaßt, welches langkettige polyungesättigte Fettsäuren als Triglyceride mit einem Verhältnis von n - 6/n - 3 von 6 bis 10 enthält.

3. Zusammensetzung nach Anspruch 2, bei der das pflanzliche Öl Sojabohnenöl ist.

4. Zusammensetzung nach Anspruch 1, bei der die Emulsion eine Teilchengröße unter 8 μm aufweist.

5. Zusammensetzung nach Anspruch 1, bei der die Emulsion eine Teilchengröße unter 4 μm aufweist, mit einer Hauptfraktion von Teilchen unter 1 μm.

6. Zusammensetzung nach Anspruch 1, bei der der Emulgator Eigelb-Phospholipide oder Sojabohnen-Phospholipide oder beide einschließt

7. Zusammensetzung nach Anspruch 1, bei der das Mittel zur Regulierung des osmotischen Druckes Glycerin einschließt.

8. Zusammensetzung nach Anspruch 1, bei der die Nährstoffe Aminosäuren oder Kohlehydrate oder beides einschließen.

**Patentansprüche für folgende Vertragsstaaten : ES, GR**

1. Verfahren zur Herstellung einer Zusammensetzung für die parenterale Ernährung, bestehend aus einer Öl-in-Wasser-Emulsion, enthaltend

a) ein Lipidgemisch von raffinierten und gereinigten pflanzlichen Ölen, enthaltend 10 bis 30 Gew.-% Öl der Gattung Borago, welches gamma-Linolensäure als Triglyceride enthält, wobei die gamma-Linolensäure 2 bis 10 Gew.-% des Lipidgemisches ausmacht,

b) L-Carnitin in einer Menge von 0,1 bis 0,5 g pro 100 g des Lipidgemisches,

c) mindestens eine Hilfskomponente, ausgewählt aus der von Emulgatoren, Stabilisierungsmitteln, Mitteln zur Regulierung des osmotischen Druckes, Konservierungsmitteln, Nährstoffen, Spurenelementen und Vitaminen gebildeten Gruppe und

d) eine wässrige Phase in einer Menge, daß die fertige Emulsion 50 bis 350 g Lipidgemisch pro 1000 ml Emulsion enthält,

dadurch gekennzeichnet, daß die Komponenten a) bis d) vermischt werden und das Gemisch anschließend homogenisiert wird.

2. Verfahren nach Anspruch 1, bei dem das Lipidgemisch weiterhin mindestens ein pflanzliches Öl umfaßt, welches langkettige polyungesättigte Fettsäuren als Triglyceride mit einem Verhältnis von n - 6/n - 3 von 6 bis 10 enthält.

3. Verfahren nach Anspruch 2, bei dem das pflanzliche Öl Sojabohnenöl ist.

4. Verfahren nach Anspruch 1, bei dem die Emulsion auf eine Teilchengröße von unter 8 μm homogenisiert wird.

5. Verfahren nach Anspruch 1, bei dem die Emulsion auf eine Teilchengröße von unter 4 μm mit einer Hauptfraktion von Teilchen unter 1 μm homogenisiert wird.

6. Verfahren nach Anspruch 1, bei dem der Emulgator Eigelb-Phospholipide oder Sojabohnen-Phospholipide oder beide einschließt.

7. Verfahren nach Anspruch 1, bei dem das Mittel zur Regulierung des osmotischen Druckes Glycerin einschließt.

8. Verfahren nach Anspruch 1, bei dem die Nährstoffe Aminosäuren oder Kohlehydrate oder beides einschließen.

**Revendications**

**Revendications pour les Etats contractants suivants : AT, BE, CH, DE, DK, FR, GB, IT, LI, LU, NL, SE**

1. Composition pour la nutrition parentérale, constituée par une émulsion huile dans l'eau, qui comprend:
   a) un mélange lipide d'huiles végétales raffinées et purifiées, contenant 10 à 30 % en poids d'huile de l'espèce bourrache, qui contient

   de l'acide gamma-linolénique en tant que triglycérides, cet acide gamma-linolénique constituant 2 à 10 % en poids du mélange lipide ;
   b) de la L-carnitine dans une quantité de 0,1 à 0,5 g pour 100 g du mélange lipide ;
   c) au moins un composant auxiliaire, choisi parmi le groupe des agents émulsifiants, des agents stabilisants, des agents pour la régulation de la pression osmotique, des agents de conservation, des agents nutritifs, des oligo-éléments et des vitamines ; et
   d) une phase aqueuse dans une quantité telle que l'émulsion terminée contient 50 à 350 g de mélange lipide pour 1000 ml d'émulsion.

2. Composition selon la revendication 1, dans laquelle le mélange lipide comprend de plus au moins une huile végétale qui contient des acides gras polyinsaturés à chaîne longue en tant que triglycérides ayant un rapport n-6/n-3 de 6 à 10.

3. Composition selon la revendication 2, dans laquelle l'huile végétale est de l'huile de soja.

4. Composition selon la revendication 1, dans laquelle l'émulsion a une granulométrie au dessous de 8 $\mu$m.

5. Composition selon la revendication 1, dans laquelle l'émulsion a une granulométrie inférieure à 4 $\mu$m avec la fraction principale de particules au dessous d'1 $\mu$m.

6. Composition selon la revendication 1, dans laquelle l'agent émulsifiant comprend des phospholipides de jaune d'oeuf ou des phospholipides de soja ou les deux à la fois.

7. Composition selon la revendication 1, dans laquelle l'agent destiné à la régulation de la pression osmotique comprend du glycérol.

8. Composition selon la revendication 1, dans laquelle les agents nutritifs comprennent des acides aminés ou des hydrates de carbone ou les deux à la fois.

**Revendications pour les Etats contractants suivants : ES, GR**

1. Procédé pour la préparation d'une composition pour la nutrition parentérale, constituée par une émulsion huile dans l'eau, qui comprend :
   a) un mélange lipide d'huiles végétales raffinées et purifiées, contenant 10 à 30 % en poids d'huile de l'espèce bourrache, qui contient

   de l'acide gamma-linolénique en tant que triglycérides, cet acide gamma-linolénique constituant 2 à 10 % en poids du mélange lipide ;
   b) de la L-carnitine dans une quantité de 0,1 a 0,5 g pour 100 g du mélange lipide ;
   c) au moins un oomposant auxiliaire, choisi parmi le groupe des agents émulsifiants, des agents stabilisants, des agents pour la régulation de la pression osmotique, des agents de conservation, des agents nutritifs, des oligo-éléments et des vitamines ; et
   d) une phase aqueuse dans une quantité telle que l'émulsion terminée contient 50 à 350 g de mélange lipide pour 1000 ml d'émulsion,

   caractérisé en ce que les composants a) jusqu'à c) sont mélangés, et le mélange est ensuite homogénéisé.

2. Procédé selon la revendication 1, dans lequel le mélange lipide comprend de plus au moins une huile végétale qui contient des acides gras polyinsaturés à chaîne longue en tant que triglycérides ayant un rapport n-6/n-3 de 6 à 10.

3. Procédé selon la revendication 2, dans lequel l'huile végétale est de l'huile de soja.

4.  Procédé selon la revendication 1, dans lequel l'émulsion est homogénéisé jusqu'à une granulométrie inférieure à 8 µm.

5.  Procédé selon la revendication 1, dans lequel l'émulsion est homogénéisé jusqu'à une granulométrie inférieure a 4 µm avec la fraction principale de particules au-dessous d'1 µm.

6.  Procédé selon la revendication 1, dans lequel l'agent émulsifiant comprend des phospholipides de jaune d'oeuf ou des phospholipides de soja ou les deux la fois.

7.  Procédé selon la revendication 1, dans lequel l'agent destiné à la régulation de la pression osmotique comprend du glycérol.

8.  Procédé selon la revendication 1, dans lequel les agents nutritifs comprennent des acides aminés ou des hydrates de carbone ou les deux à la fois.

# Biosynthesis of n-6 and n-3 fatty acids

**n-6 group**              **n-3 group**

**C 18:2** *linoleic*        **C 18:3** *α-linolenic*

← $\Delta$ 6-desaturase →

**C 18:3** *γ-linolenic*        **C 18:4**

**C 20:3** *dihomo-γ-linolenic* ①     *PGE-1 series* ←     **C 20:4**

← $\Delta$ 5-desaturase →

*PGE-2 series* ← **C 20:4** *arachidonic*      **C 20:5** *timnodonic* ④ → *PGE-3 series*

**C 22:4** *adrenic* ②        **C 22:5** ③

← $\Delta$ 4-desaturase →

**C 22:5** ③        **C 22:6** *clupanodonic* ⑤

①   Eicosatrienoic acid
②   Docosatetraenoic acid
③   Docosapentaenoic acid
④   Eicosapentaenoic acid (EPA)
⑤   Docosahexaenoic acid (DHA)

EP 0 465 550 B1